# EUROPÄISCHE PATENTANMELDUNG

(11) **EP 3 629 332 A1**
(43) Veröffentlichungstag der Anmeldung: **01.04.2020**
(21) Anmeldenummer: 18197422.1
(22) Anmeldetag: 28.09.2018
(51) Int. Cl.: G16H 10/60, G16H 20/13, G16H 20/17

(54) **SICHERES AUSGEBEN EINER SUBSTANZ**

(71) Anmelder: Siemens Aktiengesellschaft, 80333 München (DE)
(72) Erfinder: Aigner, Tobias, 81543 München (DE); Sauer, Markus, 81739 München (DE); Zupan, Nejc, 80337 München (DE)

(57) **Zusammenfassung**

Die Erfindung betrifft ein Ausgeben mindestens einer Substanz. Dazu wird ein Ausgabeplan in einer verteilten Datenbank (10) gespeichert. Der Ausgabeplan definiert, welche der mindestens einen Substanz zu welchem Zeitpunkt und in welcher Menge auszugeben ist. Die mindestens eine Substanz wird abhängig von dem Ausgabeplan ausgegeben.

## Beschreibung

### TECHNISCHES GEBIET

Die vorliegende Erfindung betrifft das sichere Ausgeben einer Substanz, um beispielsweise einen Ausgabeplan zur Verabreichung von Arzneimitteln für einen Patienten sicher und gegebenenfalls automatisch zu befolgen.

### HINTERGRUND

Aus dem Stand der Technik sind das Dokument US 8,531,247 B2, das Dokument US 8,892,616 B2, das Dokument US 8,300,811 B2, das Dokument US 9,147,088 B2, das Dokument US 9584311 B2, das Dokument EP 2976707 B1, das Dokument EP 2 605 445 B1, das Dokument EP 2 870 565 A1, das Dokument EP 2 891 102 A1, das Dokument WO 2017137256 A1, das Dokument EP 2870565 B1, EP 2891266 B1, das Dokument EP 2961091 A1, das Dokument EP 2961093 A1, das Dokument EP 3028140 B1, das Dokument EP 2930610 B1, das Dokument EP 2940620 B1, das Dokument EP 2899714 A1, das Dokument EP 2981926 A0, das Dokument EP 3125492 B1, das Dokument EP17195125, das Dokument EP17211075, das Dokument EP18178316, das Dokument EP18156084, das Dokument EP18151345, das Dokument EP18167702, das Dokument EP18153594, das Dokument EP18162050, das Dokument EP18178498, das Dokument EP18152117, das Dokument EP18150133, das Dokument EP18169145, das Dokument EP17210647, das Dokument EP18150146, das Dokument EP18167486, das Dokument EP18168670, das Dokument EP18162049, das Dokument EP17203819, das Dokument EP18157168, das Dokument EP18169421, das Dokument EP17210253, das Dokument EP17205224, das Dokument EP18169269, das Dokument EP18169254, das Dokument EP17210288, das Dokument EP18153025, das Dokument EP17200614, das Dokument EP18156308, das Dokument EP17201758, das Dokument EP18156511, das Dokument EP18159485, das Dokument EP17203573, das Dokument EP 17175275, das Dokument EP17186826, das Dokument WO 2018069271 A1, das Dokument PCT/EP2017/082508, das Dokument EP17179823, das Dokument WO 2017050348 A1, das Dokument WO 2018068965 A1 und das Dokument US 8 843 761 B2 bekannt.

Verteilungs- oder Ausgabe-Verfahren werden typischerweise mittels Medienbrüchen (engl. media breaches) ausgeführt, was fehleranfällig sein kann. Diese typischen Verteilungs- oder Ausgabe-Verfahren mit Medienbrüchen (d.h. Einsatz verschiedener Medien mit entsprechenden Unterbrechungen, z.B. in zeitlicher und/oder sachlicher Hinsicht) sollen an folgendem Beispiel näher erläutert werden. Nachfolgend wird ein illustratives Beispiel beschrieben:
Ein Arzt übergibt beispielsweise einem Patienten eine Beschreibung in Papierform, welche zahlreiche Hinweise enthält, wie Arzneimittel einzunehmen sind. Der Patient versucht, das richtige Arzneimittel bei einer Apotheke zu kaufen, in welcher er weitere Hinweise erhält, wie die Arzneimittel einzunehmen sind. Schließlich nimmt der Patient die Arzneimittel ein, wobei er die erhaltenen Hinweise zu berücksichtigen versucht. Ein Problem bei diesem beispielhaften Vorgehen ist, dass es unklar bleibt und nicht nachvollziehbar ist, ob bei der Einnahme der Arzneimittel die erhaltenen Hinweise auch berücksichtigt wurden und wer welche Hinweise gegeben hat.

### ZUSAMMENFASSUNG

Daher stellt sich die vorliegende Erfindung die Aufgabe, das Ausgeben einer Substanz derart zu realisieren, dass Hinweise oder Bedingungen zur Ausgabe der Substanz bei der Ausgabe der Substanz sicher berücksichtigt werden.

Erfindungsgemäß wird diese Aufgabe durch ein Verfahren zum Ausgeben mindestens einer Substanz nach Anspruch 1, durch eine Vorrichtung zum Ausgeben mindestens einer Substanz nach Anspruch 8, durch ein System zum Ausgeben mindestens einer Substanz nach Anspruch 12 und durch ein Computerprogrammprodukt nach Anspruch 15 gelöst.

Die Technologie von Blockketten (engl. Blockchains) bzw. "Distributed Ledgers" ist aktuell eine intensiv diskutierte Technologie, die insbesondere als verteiltes Datenbanksystem realisiert sein kann. Neben Anwendungen für dezentrale Bezahlsysteme (z. B. Bitcoin) werden in der Finanzindustrie neue Anwendungsmöglichkeiten entwickelt. Insbesondere können Transaktionen zwischen Firmen dadurch ohne Vermittler bzw. Clearing-Stelle manipulationsgeschützt realisiert werden. Dies ermöglicht neue Geschäftsmodelle ohne einen vertrauenswürdigen Vermittler, es reduziert die Transaktionskosten, und es können flexibel neue digitale Dienste angeboten werden, ohne eine dafür speziell eingerichtete Infrastruktur und Vertrauensbeziehungen einrichten zu müssen. Ein durch eine Blockkette geschützter Transaktionsdatensatz (oder kurz Transaktion) umfasst z. B. Programmcode, der auch als sogenannter "Smart Contract" bezeichnet werden kann.

Sofern es in der nachfolgenden Beschreibung nicht anders angegeben ist, beziehen sich die Begriffe "durchführen", "berechnen", "rechnergestützt", "rechnen", "feststellen", "generieren", "konfigurieren", "rekonstruieren", "steuern", "zuweisen" und dergleichen vorzugsweise auf Handlungen und/oder Prozesse und/oder Verarbeitungsschritte, die Daten verändern und/oder erzeugen und/oder die Daten in andere Daten überführen, wobei die Daten insbesondere als physikalische Größen dargestellt werden oder vorliegen können, beispielsweise als elektrische Impulse. Insbesondere sollte der Ausdruck "Computer" oder "Vorrichtung" möglichst breit ausgelegt werden, um insbesondere alle elektronischen Geräte mit Datenverarbeitungseigenschaften abzudecken. Computer können somit beispielsweise Personal Computer, Server, speicherprogrammierbare Steuerungen (SPS), Handheld-Computer-Systeme, Pocket-PC-Geräte, Mobilfunkgeräte und andere Kommunikationsgeräte, die rechnergestützt Daten verarbeiten können, Prozessoren und andere elektronische Geräte zur Datenverarbeitung sein.

Unter "rechnergestützt" oder "automatisch" kann im Zusammenhang mit der Erfindung beispielsweise eine Implementierung des Verfahrens verstanden werden, bei dem insbesondere ein Prozessor mindestens einen Verfahrensschritt des Verfahrens ausführt.

Unter einem Prozessor kann im Zusammenhang mit der Erfindung beispielsweise eine Maschine oder eine elektronische Schaltung verstanden werden. Bei einem Prozessor kann es sich insbesondere um einen Hauptprozessor (engl. Central Processing Unit, CPU), einen Mikroprozessor oder einen Mikrokontroller, beispielsweise eine anwendungsspezifische integrierte Schaltung oder einen digitalen Signalprozessor, möglicherweise in Kombination mit einer Speichereinheit zum Speichern von Programmbefehlen, etc. handeln. Bei einem Prozessor kann es sich beispielsweise auch um einen IC (integrierter Schaltkreis, engl. Integrated Circuit), insbesondere einen FPGA (engl. Field Programmable Gate Array) oder einen ASIC (anwendungsspezifische integrierte Schaltung, engl. Application-Specific Integrated Circuit), oder einen DSP (Digitaler Signalprozessor, engl. Digital Signal Processor) oder einen Grafikprozessor GPU (Graphic Processing Unit) handeln. Auch kann unter einem Prozessor ein virtualisierter Prozessor, eine virtuelle Maschine oder eine Soft-CPU verstanden werden. Es kann sich beispielsweise auch um einen programmierbaren Prozessor handeln, der mit Konfigurationsschritten zur Ausführung des genannten erfindungsgemäßen Verfahrens ausgerüstet wird oder mit Konfigurationsschritten derart konfiguriert ist, dass der programmierbare Prozessor die erfindungsgemäßen Merkmale des Verfahrens, der Komponente, der Module, oder anderer Aspekte und/oder Teilaspekte der Erfindung realisiert.

Unter einer "Speichereinheit" oder "Speichermodul" und dergleichen kann im Zusammenhang mit der Erfindung beispielsweise ein flüchtiger Speicher in Form von Arbeitsspeicher (engl. Random-Access Memory, RAM) oder ein dauerhafter Speicher wie eine Festplatte oder ein Datenträger verstanden werden. Unter einem "Modul" oder einer "Recheneinheit" kann im Zusammenhang mit der Erfindung beispielsweise ein Prozessor und/oder eine Speichereinheit zum Speichern von Programmbefehlen verstanden werden. Beispielsweise ist der Prozessor speziell dazu eingerichtet, die Programmbefehle derart auszuführen, damit der Prozessor Funktionen ausführt, um das erfindungsgemäße Verfahren oder einen Schritt des erfindungsgemäßen Verfahrens zu implementieren oder realisieren. Ein Modul kann beispielsweise auch ein Knoten des verteilten Datenbanksystems sein, der beispielsweise die spezifischen Funktionen/Merkmale eines entsprechenden Moduls realisiert. Die jeweiligen Module können beispielsweise auch als separate bzw. eigenständige Module ausgebildet sein. Hierzu können die entsprechenden Module beispielsweise weitere Elemente umfassen. Diese Elemente sind beispielsweise eine oder mehrere Schnittstellen (z. B. Datenbankschnittstellen, Kommunikationsschnittstellen - z. B. Netzwerkschnittstelle, WLAN-Schnittstelle) und/oder eine Evaluierungseinheit (z. B. ein Prozessor) und/oder eine Speichereinheit. Mittels der Schnittstellen können beispielsweise Daten ausgetauscht (z. B. empfangen, übermittelt, gesendet oder bereitgestellt werden). Mittels der Evaluierungseinheit können Daten beispielsweise rechnergestützt und/oder automatisiert verglichen, überprüft, verarbeitet, zugeordnet oder berechnet werden. Mittels der Speichereinheit können Daten beispielsweise rechnergestützt und/oder automatisiert gespeichert, abgerufen oder bereitgestellt werden.

Unter "umfassen", insbesondere in Bezug auf Daten und/oder Informationen, kann im Zusammenhang mit der Erfindung beispielsweise ein (rechnergestütztes) Speichern einer entsprechenden Information bzw. eines entsprechenden Datums in einer Datenstruktur/Datensatz (die z. B. wiederum in einer Speichereinheit gespeichert ist) verstanden werden.

Unter "zuordnen", insbesondere in Bezug auf Daten und/oder Informationen, kann im Zusammenhang mit der Erfindung beispielsweise eine rechnergestützte Zuordnung von Daten und/oder Informationen verstanden werden. Beispielsweise wird einem ersten Datum hierzu mittels einer Speicheradresse oder eines eindeutigen Identifizierers (engl. unique identifier (UID)) ein zweites Datum zugeordnet, in dem z. B. das erste Datum zusammen mit der Speicheradresse oder des eindeutigen Identifizierers des zweiten Datums zusammen in einem Datensatz gespeichert wird.

Unter "bereitstellen", insbesondere in Bezug auf Daten und/oder Informationen, kann im Zusammenhang mit der Erfindung beispielsweise ein rechnergestütztes Bereitstellen verstanden werden. Das Bereitstellen erfolgt beispielsweise über eine Schnittstelle (z. B. eine Datenbankschnittstelle, eine Netzwerkschnittstelle, eine Schnittstelle zu einer Speichereinheit). Über diese Schnittstelle können beispielsweise beim Bereitstellen entsprechende Daten und/oder Informationen übermittelt und/oder gesendet und/oder abgerufen und/oder empfangen werden.

Unter "bereitstellen" kann im Zusammenhang mit der Erfindung beispielsweise auch ein Laden oder ein Speichern, beispielsweise einer Transaktion mit entsprechenden Daten verstanden werden. Dies kann beispielsweise auf oder von einem Speichermodul erfolgen. Unter "Bereitstellen" kann beispielsweise auch ein Übertragen (oder ein Senden oder ein Übermitteln) von entsprechenden Daten von einem Knoten zu einem anderen Knoten der Blockkette oder des verteilten Datenbanksystems (bzw. deren Infrastruktur) verstanden werden.

Unter "Smart-Contract-Prozess" kann im Zusammenhang mit der Erfindung insbesondere ein Ausführen eines Programmcodes (z. B. der Steuerbefehle) in einem Prozess durch das verteilte Datenbanksystem bzw. deren Infrastruktur verstanden werden.

Unter einer "Prüfsumme", beispielsweise eine Datenblockprüfsumme, eine Datenprüfsumme, eine Knotenprüfsumme, eine Transaktionsprüfsumme, eine Verkettungsprüfsumme oder dergleichen, kann im Zusammenhang mit der Erfindung beispielsweise eine kryptographische Prüfsumme oder kryptographischer Hash bzw. Hashwert verstanden werden, die insbesondere mittels einer kryptographischen Hashfunktion über einen Datensatz und/oder Daten und/oder eine oder mehrere der Transaktionen und/oder einem Teilbereich eines Datenblocks (z. B. der Block-Header eines Blocks einer Blockkette oder Datenblock-Header eines Datenblocks des verteilten Datenbanksystems oder nur einem Teil der Transaktionen eines Datenblocks) gebildet oder berechnet werden. Bei einer Prüfsumme kann es sich insbesondere um eine Prüfsumme/n oder Hashwert/e eines Hash-Baumes (z. B. Merkle Baum, Patricia-Baum) handeln. Weiterhin kann darunter insbesondere auch eine digitale Signatur oder ein kryptographischer Nachrichtenauthentisierungscode verstanden werden. Mittels der Prüfsummen kann beispielsweise auf unterschiedlichen Ebenen des Datenbanksystems ein kryptographischer Schutz/Manipulationsschutz für die Transaktionen und die darin gespeicherten Daten(sätze) realisiert werden. Ist beispielsweise eine hohe Sicherheit gefordert, werden beispielsweise die Prüfsummen auf Transaktionsebene erzeugt und überprüft. Ist eine weniger hohe Sicherheit gefordert, werden beispielsweise die Prüfsummen auf Blockebene (z. B. über den ganzen Datenblock oder nur über einen Teil des Datenblocks und/oder einen Teil der Transkationen) erzeugt und überprüft.

Unter einer "Datenblockprüfsumme" kann im Zusammenhang mit der Erfindung eine Prüfsumme verstanden werden, die beispielsweise über einen Teil oder alle Transaktionen eines Datenblocks berechnet wird. Ein Knoten kann dann beispielsweise die Integrität/Authentizität des entsprechenden Teils eines Datenblocks mittels der Datenblockprüfsumme prüfen/feststellen. Zusätzlich oder alternativ kann die Datenblockprüfsumme insbesondere auch über Transaktionen eines vorhergehenden Datenblocks/Vorgänger-Datenblocks des Datenblocks gebildet worden sein. Die Datenblockprüfsumme kann dabei insbesondere auch mittels eines Hash-Baumes, beispielsweise einem Merkle Baum [1] oder einem Patricia-Baum, realisiert werden, wobei die Datenblockprüfsumme insbesondere die Wurzel-Prüfsumme des Merkle-Baumes bzw. eines Patricia-Baumes bzw. eines binären Hashbaumes ist. Insbesondere werden Transaktionen mittels weiterer Prüfsummen aus dem Merkle-Baum bzw. Patricia-Baum abgesichert (z. B. unter Verwendung der Transaktionsprüfsummen), wobei insbesondere die weiteren Prüfsummen Blätter im Merkle-Baum bzw. Patricia-Baum sind. Die Datenblockprüfsumme kann damit beispielsweise die Transaktionen absichern, indem die Wurzel-Prüfsumme aus den weiteren Prüfsummen gebildet wird. Die Datenblockprüfsumme kann insbesondere für Transaktionen eines bestimmten Datenblocks der Datenblöcke berechnet werden. Insbesondere kann eine solche Datenblockprüfsumme in einen nachfolgenden Datenblock des bestimmten Datenblocks eingehen, um diesen nachfolgenden Datenblock beispielsweise mit seinen vorhergehenden Datenblöcken zu verketten und insbesondere damit eine Integrität des verteilten Datenbanksystems prüfbar zu machen. Hierdurch kann die Datenblockprüfsumme beispielsweise die Funktion der Verkettungsprüfsumme übernehmen oder in die Verkettungsprüfsumme eingehen. Der Header eines Datenblocks (z. B. eines neuen Datenblocks oder des Datenblocks für den die Datenblockprüfsumme gebildet wurde) kann beispielsweise die Datenblockprüfsumme umfassen.

Unter "Transaktionsprüfsumme" kann im Zusammenhang mit der Erfindung eine Prüfsumme verstanden werden, die insbesondere über eine Transaktion eines Datenblocks gebildet wird. Zusätzlich kann beispielsweise eine Berechnung einer Datenblockprüfsumme für einen entsprechenden Datenblock beschleunigt werden, da hierfür beispielsweise bereits berechnete Transaktionsprüfsummen gleich als Blätter z. B. eines Merkle-Baumes verwendet werden können.

Unter einer "Verkettungsprüfsumme" kann im Zusammenhang mit der Erfindung eine Prüfsumme verstanden werden, die insbesondere einen jeweiligen Datenblock des verteilten Datenbanksystems den vorhergehenden Datenblock des verteilten Datenbanksystems angibt bzw. referenziert (in der Fachliteratur insbesondere häufig als "previous block hash" bezeichnet) [1]. Hierfür wird insbesondere für den entsprechenden vorhergehenden Datenblock eine entsprechende Verkettungsprüfsumme gebildet. Als Verkettungsprüfsumme kann beispielsweise eine Transaktionsprüfsumme oder die Datenblockprüfsumme eines Datenblocks (also ein vorhandener Datenblock des verteilten Datenbanksystems) verwendet werden, um einen neuen Datenblock mit einem (vorhandenen) Datenblock des verteilten Datenbanksystems zu verketten. Es ist beispielsweise aber auch möglich, dass eine Prüfsumme über einen Header des vorhergehenden Datenblocks oder über den gesamten vorhergehenden Datenblock gebildet wird und als Verkettungsprüfsumme verwendet wird. Dies kann beispielsweise auch für mehrere oder alle vorhergehenden Datenblöcke berechnet werden. Es ist beispielsweise auch realisierbar, dass über den Header eines Datenblocks und der Datenblockprüfsumme die Verkettungsprüfsumme gebildet wird. Ein jeweiliger Datenblock des verteilten Datenbanksystems umfasst jedoch vorzugsweise jeweils eine Verkettungsprüfsumme, die für einen vorhergehenden Datenblock, insbesondere noch bevorzugter den direkt vorhergehenden Datenblock, des jeweiligen Datenblockes berechnet wurde bzw. sich auf diesen beziehen. Es ist beispielsweise auch möglich, dass eine entsprechende Verkettungsprüfsumme auch nur über einen Teil des entsprechenden Datenblocks (z. B. vorhergehenden Datenblock) gebildet wird. Hierdurch kann beispielsweise ein Datenblock realisiert werden, der einen integritätsgeschützten Teil und einen ungeschützten Teil umfasst. Damit ließe sich beispielsweise ein Datenblock realisieren, dessen integritätsgeschützter Teil unveränderlich ist und dessen ungeschützter Teil auch noch später verändert werden kann. Unter integritätsgeschützt ist dabei insbesondere zu verstehen, dass eine Veränderung von integritätsgeschützten Daten mittels einer Prüfsumme feststellbar ist.

Die Daten, die beispielsweise in einer Transkation eines Datenblocks gespeichert werden, können insbesondere auf unterschiedliche Weise bereitgestellt werden. Anstelle der Daten, z. B. Nutzerdaten wie Messdaten oder Daten/Eigentumsverhältnisse zu Assets, kann beispielsweise eine Transaktion eines Datenblocks nur die Prüfsumme für diese Daten umfassen. Die entsprechende Prüfsumme kann dabei auf unterschiedliche Weise realisiert werden. Dies kann z. B. eine entsprechende Datenblockprüfsumme eines Datenblocks (mit den entsprechenden Daten) einer anderen Datenbank oder des verteilten Datenbanksystems sein, eine Transaktionsprüfsumme eines Datenblocks mit den entsprechenden Daten (des verteilten Datenbanksystems oder einer anderen Datenbank) oder eine Datenprüfsumme, die über die Daten gebildet wurde.

Zusätzlich kann die entsprechende Transkation noch einen Verweis oder eine Angabe zu einem Speicherort (z. B. eine Adresse eines Fileservers und Angaben, wo die entsprechenden Daten auf dem Fileserver zu finden sind; oder eine Adresse einer anderen verteilten Datenbank, welche die Daten umfasst) umfassen. Die entsprechenden Daten könnten dann beispielsweise auch in einer weiteren Transaktion eines weiteren Datenblocks des verteilten Datenbanksystems bereitgestellt werden (z. B. wenn die entsprechenden Daten und die zugehörigen Prüfsummen in unterschiedlichen Datenblöcken umfasst sind). Es ist beispielsweise aber auch denkbar, dass diese Daten über einen anderen Kommunikationskanal (z. B. über eine andere Datenbank und/oder einen kryptographisch gesicherten Kommunikationskanal) bereitgestellt werden.

Auch kann beispielsweise zusätzlich zu der Prüfsumme ein Zusatzdatensatz (z. B. ein Verweis oder eine Angabe zu einem Speicherort) in den entsprechenden Transaktionen abgelegt sein, der insbesondere einen Speicherort angibt, wo die Daten abgerufen werden können. Das ist insbesondere dahingehend vorteilhaft, um eine Datengröße der Blockkette oder des verteilten Datenbanksystems möglichst gering zu halten.

Unter "sicherheitsgeschützt" oder einer "Sicherheit von Daten" kann im Zusammenhang mit der Erfindung beispielsweise ein Schutz verstanden werden, der insbesondere durch ein kryptographisches Verfahren realisiert wird. Beispielsweise kann dies durch eine Nutzung des verteilten Datenbanksystems für das Bereitstellen oder Übertragen oder Senden von entsprechenden Daten/Transaktionen realisiert werden. Dies wird vorzugsweise durch eine Kombination der verschiedenen (kryptographischen) Prüfsummen erreicht, indem diese insbesondere synergetisch zusammenwirken, um beispielsweise die Sicherheit bzw. die kryptographische Sicherheit für die Daten der Transaktionen zu verbessern. Mit anderen Worten kann insbesondere unter "sicherheitsgeschützt" im Zusammenhang mit der Erfindung auch "kryptographisch geschützt" und/oder "manipulationsgeschützt" verstanden werden, wobei "manipulationsgeschützt" auch als "integritätsgeschützt" bezeichnet werden kann.

Unter "Verketten der/von Datenblöcken eines verteilten Datenbanksystems" kann im Zusammenhang mit der Erfindung beispielsweise verstanden werden, dass Datenblöcke jeweils eine Information (z. B. Verkettungsprüfsumme) umfassen, die auf einen anderen Datenblock oder mehrere andere Datenblöcke des verteilten Datenbanksystems verweisen bzw. diese referenzieren [1] [4] [5] .

Unter "Einfügen in das verteilte Datenbanksystem", "Speichern in der verteilten Datenbank" oder "Hinterlegen von Daten in der verteilten Datenbank" und dergleichen kann im Zusammenhang mit der Erfindung beispielsweise verstanden werden, dass insbesondere eine Transaktion bzw. die Transaktionen oder ein Datenblock mit seinen Transaktionen an einen oder mehrere Knoten eines verteilten Datenbanksystems übermittelt wird. Werden diese Transaktionen beispielsweise erfolgreich validiert (z. B. durch den/die Knoten), werden diese Transaktionen insbesondere als neuer Datenblock mit mindestens einem vorhandenen Datenblock des verteilten Datenbanksystems verkettet [1][4][5]. Hierzu werden die entsprechenden Transaktionen beispielsweise in einem neuen Datenblock gespeichert. Insbesondere kann dieses Validieren und/oder Verketten durch einen vertrauenswürdigen Knoten (z. B. einen Mining Node, einen Blockketten-Orakel oder eine Blockketten-Plattform) erfolgen. Insbesondere kann dabei unter einer Blockketten-Plattform eine Blockkette als Dienst (engl. Blockkette als Service) verstanden werden, wie dies insbesondere durch Microsoft oder IBM vorgeschlagen wird. Insbesondere können ein vertrauenswürdiger Knoten und/oder ein Knoten jeweils eine Knoten-Prüfsumme (z. B. eine digitale Signatur) in einem Datenblock hinterlegen (z. B. in denen von ihnen validierten und erzeugten Datenblock, der dann verkettet wird), um insbesondere eine Identifizierbarkeit des Erstellers des Datenblockes zu ermöglichen und/oder eine Identifizierbarkeit des Knotens zu ermöglichen. Dabei gibt diese Knoten-Prüfsumme an, welcher Knoten beispielsweise den entsprechenden Datenblock mit mindestens einem anderen Datenblock des verteilten Datenbanksystems verkettet hat.

Unter "Transaktion" bzw. "Transaktionen" können im Zusammenhang mit der Erfindung beispielsweise ein Smart-Contract [4] [5], eine Datenstruktur oder ein Transaktionsdatensatz verstanden werden, der insbesondere jeweils eine der Transaktionen oder mehrere Transkationen umfasst. Unter "Transaktion" bzw. "Transaktionen" können im Zusammenhang mit der Erfindung beispielsweise auch die Daten einer Transaktion eines Datenblocks einer Blockkette (engl. Blockchain) verstanden werden. Eine Transaktion kann insbesondere einen Programmcode umfassen, der beispielsweise einen Smart Contract realisiert. Beispielsweise können im Zusammenhang mit der Erfindung unter Transaktion auch eine Steuertransaktionen und/oder Bestätigungstransaktion verstanden werden. Alternativ kann eine Transaktion beispielsweise eine Datenstruktur sein, die Daten speichert (z. B. die Steuerbefehle und/oder Vertragsdaten und/oder andere Daten wie Videodaten, Nutzerdaten, Messdaten etc.).

Insbesondere ist unter "Speichern von Transaktionen in Datenblöcken", "Speichern von Transaktionen" und dergleichen ein direktes Speichern oder indirektes Speichern zu verstehen. Unter einem direkten Speichern kann dabei beispielsweise verstanden werden, dass der entsprechende Datenblock (des verteilten Datenbanksystems) oder die entsprechende Transaktion des verteilten Datenbanksystems) die jeweiligen Daten umfasst. Unter einem indirekten Speichern kann dabei beispielsweise verstanden werden, dass der entsprechende Datenblock oder die entsprechende Transaktion eine Prüfsumme und optional einen Zusatzdatensatz (z. B. einen Verweis oder eine Angabe zu einem Speicherort) für entsprechende Daten umfasst und die entsprechenden Daten somit nicht direkt in dem Datenblock (oder der Transaktion) gespeichert sind (also stattdessen nur eine Prüfsumme für diese Daten). Insbesondere können beim Speichern von Transaktionen in Datenblöcken diese Prüfsummen beispielsweise validiert werden, so wie dies beispielsweise unter "Einfügen in das verteilte Datenbanksystem" erläutert ist.

Unter einem "Programmcode" (z. B. ein Smart-Contract) kann im Zusammenhang mit der Erfindung beispielsweise ein Programmbefehl oder mehrere Programmbefehle verstanden werden, die insbesondere in einer oder mehreren Transaktionen gespeichert sind. Der Programmcode ist insbesondere ausführbar und wird beispielsweise durch das verteilte Datenbanksystem ausgeführt. Dies kann beispielsweise mittels einer Ausführungsumgebung (z. B. einer virtuellen Maschine) realisiert werden, wobei die Ausführungsumgebung bzw. der Programmcode vorzugsweise Turing-vollständig sind. Der Programmcode wird vorzugsweise durch die Infrastruktur des verteilten Datenbanksystems ausgeführt [4][5]. Dabei wird beispielsweise eine virtuelle Maschine durch die Infrastruktur des verteilten Datenbanksystems realisiert.

Unter einem "Smart Contract" kann im Zusammenhang mit der Erfindung beispielsweise ein ausführbarer Programmcode verstanden werden [4][5] (siehe insbesondere Definition "Programmcode"). Der Smart Contract ist vorzugsweise in einer Transaktion eines verteilten Datenbanksystems (z. B. eine Blockkette) gespeichert, beispielsweise in einem Datenblock des verteilten Datenbanksystems. Beispielsweise kann der Smart Contract auf die gleiche Weise ausgeführt werden, wie dies bei der Definition von "Programmcode", insbesondere im Zusammenhang mit der Erfindung, erläutert ist.

Unter "Proof-of-Work-Nachweis" kann im Zusammenhang mit der Erfindung beispielsweise ein Lösen einer rechenintensiven Aufgabe verstanden werden, die insbesondere abhängig vom Datenblock-Inhalt/Inhalt einer bestimmten Transaktion zu lösen ist [1][4][5]. Eine solche rechenintensive Aufgabe wird beispielsweise auch als kryptographisches Puzzle bezeichnet.

Unter einem "verteilten Datenbanksystem", das beispielsweise auch als verteilte Datenbank bezeichnet werden kann, kann im Zusammenhang mit der Erfindung beispielsweise eine dezentral verteilte Datenbank, eine Blockkette (engl. Blockchain), ein distributed Ledger, ein verteiltes Speichersystem, ein distributed ledger technology (DLT) based system (DLTS), ein revisionssicheres Datenbanksystem, eine Cloud, ein Cloud-Service, eine Blockkette in einer Cloud oder eine Peer-to-Peer Datenbank verstanden werden. Auch können beispielsweise unterschiedliche Implementierungen einer Blockkette oder eines DLTS verwendet werden, wie z. B. eine Blockkette oder ein DLTS, die mittels eines Directed Acylic Graph (DAG), eines kryptographischen Puzzles, einem Hashgraph oder eine Kombination aus den genannten Implementierungsvarianten [6][7]. Auch können beispielsweise unterschiedliche Konsensusverfahren (engl. consensus algorithms) implementiert werden. Dies kann beispielsweise ein Konsensusverfahren mittels eines kryptographischen Puzzles, Gossip about Gossip, Virtual Voting oder eine Kombination der genannten Verfahren sein (z. B. Gossip about Gossip kombiniert mit Virtual Voting) [6][7]. Wird beispielsweise eine Blockkette verwendet, so kann diese insbesondere mittels einer Bitcoin-basierten Realisierung oder einer Ethereum-basierten Realisierung umgesetzt werden [1][4][5]. Unter einem "verteilten Datenbanksystem" kann beispielsweise auch ein verteiltes Datenbanksystem verstanden werden, von dem zumindest ein Teil seiner Knoten und/oder Geräte und/oder Infrastruktur durch eine Cloud realisiert sind. Beispielsweise sind die entsprechenden Komponenten als Knoten/Geräte in der Cloud (z. B. als virtueller Knoten in einer virtuellen Maschine) realisiert. Dies kann beispielsweise mittels VM-Ware, Amazon Web Services oder Microsoft Azure erfolgen. Aufgrund der hohen Flexibilität der erläuterten Implementierungsvarianten, können insbesondere auch Teilaspekte der genannten Implementierungsvarianten miteinander kombiniert werden, indem z. B. ein Hashgraph als Blockkette verwendet wird, wobei die Blockkette selbst z. B. auch blocklos sein kann.

Wird beispielsweise ein Directed Acylic Graph (DAG) verwendet (z. B. IOTA oder Tangle), sind insbesondere Transaktionen oder Blöcke oder Knoten des Graphen miteinander über gerichtete Kanten miteinander verbunden. Dies bedeutet insbesondere, dass (alle) Kanten (immer) die gleiche Richtung haben, ähnlich wie dies z. B. bei Zeit ist. Mit anderen Worten ist es insbesondere nicht möglich rückwärts (also entgegen der gemeinsamen gleichen Richtung) die Transaktionen oder die Blöcke oder die Knoten des Graphen anzulaufen bzw. anzuspringen. Azyklisch bedeutet dabei insbesondere, dass es keine Schleifen bei einem Durchlaufen des Graphen gibt.

Bei dem verteilten Datenbanksystem kann es sich beispielsweise um ein öffentliches verteiltes Datenbanksystem (z. B. eine öffentliche Blockkette) oder ein geschlossenes (oder privates) verteiltes Datenbanksystem (z. B. eine private Blockkette) handeln.

Handelt es sich beispielsweise um ein öffentliches verteiltes Datenbanksystem, bedeutet dies, dass neue Knoten und/oder Geräte ohne Berechtigungsnachweise oder ohne Authentifizierung oder ohne Anmeldeinformationen oder ohne Credentials dem verteilten Datenbanksystem beitreten können bzw. von diesem akzeptiert werden. Insbesondere können in einem solchen Fall die Betreiber der Knoten und/oder Geräte anonym bleiben.

Handelt es sich bei dem verteilten Datenbanksystem beispielsweise um ein geschlossenes verteiltes Datenbanksystem, benötigen neue Knoten und/oder Geräte beispielsweise einen gültigen Berechtigungsnachweis und/oder gültige Authentifizierungsinformationen und/oder gültige Credentials und/oder gültige Anmeldeinformationen, um dem verteilten Datenbanksystem beitreten können bzw. von diesem akzeptiert zu werden.

Bei einem verteilten Datenbanksystem kann es sich beispielsweise auch um ein verteiltes Kommunikationssystem zum Datenaustausch handeln. Dies kann beispielsweise ein Netzwerk oder ein Peer-2-Peer Netzwerk sein.

Unter "Datenblock", der insbesondere je nach Kontext und Realisierung auch als "Glied" oder "Block" bezeichnet sein kann, kann im Zusammenhang mit der Erfindung beispielsweise ein Datenblock eines verteilten Datenbanksystems (z. B. eine Blockkette oder eine Peer to Peer Datenbank) verstanden werden, die insbesondere als Datenstruktur realisiert ist und vorzugsweise jeweils eine der Transaktionen oder mehrere der Transaktionen umfasst. Bei einer Implementierung kann beispielsweise die Datenbank (oder das Datenbanksystem) ein DLT basiertes System (DLTS) oder eine Blockkette sein und ein Datenblock ein Block der Blockkette oder des DLTS. Ein Datenblock kann beispielsweise Angaben zur Größe (Datengröße in Byte) des Datenblocks, einen Datenblock-Header (engl. Block-header), einen Transaktionszähler und eine oder mehrere Transaktionen umfassen [1]. Der Datenblock-Header kann beispielsweise eine Version, eine Verkettungsprüfsumme, eine Datenblockprüfsumme, einen Zeitstempel, einen Proof-of-Work Nachweis und eine Nonce (Einmalwert, Zufallswert oder Zähler, der für den Proof-of-Work Nachweis verwendet wird) umfassen [1][4][5]. Bei einem Datenblock kann es sich beispielsweise auch nur um einen bestimmten Speicherbereich oder Adressbereich der Gesamtdaten handeln, die in dem verteilten Datenbanksystem gespeichert sind. Damit lassen sich beispielsweise blocklose (engl. blockless) verteilte Datenbanksysteme, wie z. B. die IoT Chain (ITC), IOTA, und Byteball, realisieren. Hierbei werden insbesondere die Funktionalitäten der Blöcke einer Blockkette und der Transaktionen miteinander derart kombiniert, dass z. B. die Transaktionen selbst die Sequenz oder Kette von Transaktionen (des verteilten Datenbanksystems) absichern (also insbesondere sicherheitsgeschützt gespeichert werden). Hierzu können beispielsweise mit einer Verkettungsprüfsumme die Transaktionen selbst miteinander verkettet werden, indem vorzugsweise eine separate Prüfsumme oder die Transaktionsprüfsumme einer oder mehrerer Transaktionen als Verkettungsprüfsumme dient, die beim Speichern einer neuen Transaktion in dem verteilten Datenbanksystem in der entsprechenden neuen Transaktion mit gespeichert wird. In einer solchen Ausführungsform kann ein Datenblock beispielsweise auch eine oder mehrere Transaktionen umfassen, wobei im einfachsten Fall beispielsweise ein Datenblock einer Transaktion entspricht.

Unter "Nonce" kann im Zusammenhang mit der Erfindung beispielsweise eine kryptographische Nonce verstanden werden (Abkürzung für: "used only once"[2] oder "number used once"[3]). Insbesondere bezeichnet eine Nonce einzelne Zahlen- oder eine Buchstabenkombination, die vorzugsweise ein einziges Mal in dem jeweiligen Kontext (z. B. Transaktion, Datenübertragung) verwendet wird.

Unter "vorhergehende Datenblöcke eines (bestimmten) Datenblockes des verteilten Datenbanksystems" kann im Zusammenhang mit der Erfindung beispielsweise der Datenblock des verteilten Datenbanksystems verstanden werden, der insbesondere einem (bestimmten) Datenblock direkt vorhergeht. Alternativ können unter "vorhergehender Datenblöcke eines (bestimmten) Datenblockes des verteilten Datenbanksystems" insbesondere auch alle Datenblöcke des verteilten Datenbanksystems verstanden werden, die dem bestimmten Datenblock vorhergehen. Hierdurch kann beispielsweise die Verkettungsprüfsumme oder die Transaktionsprüfsumme insbesondere nur über das dem bestimmten Datenblock direkt vorhergehenden Datenblock (bzw. deren Transaktionen) oder über alle dem ersten Datenblock vorhergehenden Datenblöcke (bzw. deren Transaktionen) gebildet werden.

Unter einem "Blockketten-Knoten", "Knoten", "Knoten eines verteilten Datenbanksystems" und dergleichen, können im Zusammenhang mit der Erfindung beispielsweise Geräte (z. B. Feldgeräte, Mobiltelefone), Rechner, Smart-Phones, Clients oder Teilnehmer verstanden werden, die Operationen (mit) dem verteilten Datenbanksystem (z. B. eine Blockkette) durchführen [1][4][5]. Solche Knoten können beispielsweise Transaktionen eines verteilten Datenbanksystems bzw. deren Datenblöcke ausführen oder neue Datenblöcke mit neuen Transaktionen in das verteilte Datenbanksystem mittels neuer Datenblöcke einfügen bzw. verketten. Insbesondere kann dieses Validieren und/oder Verketten durch einen vertrauenswürdigen Knoten (z. B. einem Mining Node) oder ausschließlich durch vertrauenswürdige Knoten erfolgen. Bei einem vertrauenswürdigen Knoten handelt es sich beispielsweise um einen Knoten, der über zusätzliche Sicherheitsmaßnahmen verfügt (z. B. Firewalls, Zugangsbeschränkungen zum Knoten oder ähnliches), um eine Manipulation des Knotens zu verhindern. Alternativ oder zusätzlich kann beispielsweise ein vertrauenswürdiger Knoten beim Verketten eines neuen Datenblocks mit dem verteilten Datenbanksystem, eine Knotenprüfsumme (z. B. eine digitale Signatur oder ein Zertifikat) in dem neuen Datenblock speichern. Damit kann insbesondere ein Nachweis bereitgestellt werden, der angibt, dass der entsprechende Datenblock von einem bestimmten Knoten eingefügt wurde bzw. seine Herkunft angibt. Bei den Geräten (z. B. dem entsprechenden Gerät) handelt es sich beispielsweise um Geräte eines technischen Systems und/oder industriellen Anlage und/oder eines Automatisierungsnetzes und/oder einer Fertigungsanlage, die insbesondere auch ein Knoten des verteilten Datenbanksystems sind. Dabei können die Geräte beispielsweise Feldgeräte sein oder Geräte im Internet der Dinge sein, die insbesondere auch ein Knoten des verteilten Datenbanksystems sind. Knoten können beispielsweise auch zumindest einen Prozessor umfassen, um z. B. ihre computerimplementierte Funktionalität auszuführen.

Unter einem "Blockketten-Orakel" und dergleichen können im Zusammenhang mit der Erfindung beispielsweise Knoten, Geräte oder Rechner verstanden werden, die z. B. über ein Sicherheitsmodul verfügen, das beispielsweise mittels Software-Schutzmechanismen (z. B. kryptographische Verfahren), mechanische Schutzeinrichtungen (z. B. ein abschließbares Gehäuse) oder elektrische Schutzeinrichtungen verfügt (z. B. Tamper-Schutz oder ein Schutzsystem, das die Daten des Sicherheitsmoduls bei einer unzulässigen Nutzung/Behandlung des Blockketten-Orakel löscht umfasst). Das Sicherheitsmodul kann dabei beispielsweise kryptographische Schlüssel umfassen, die für die Berechnung der Prüfsummen (z. B. Transaktionsprüfsummen oder Knotenprüfsummen) notwendig sind.

Unter einem "Rechner", einer "Vorrichtung" oder einem "Gerät" kann im Zusammenhang mit der Erfindung beispielsweise ein Computer(system), ein Client, ein Smart-Phone, ein Gerät oder ein Server, die jeweils außerhalb der Blockkette angeordnet sind bzw. kein Teilnehmer des verteilten Datenbanksystems (z. B. der Blockkette) sind (also keine Operationen mit dem verteilten Datenbanksystem durchführen oder diese nur abfragen, ohne jedoch Transaktionen durchzuführen, Datenblöcke einfügen oder Proof-of-Work-Nachweise berechnen), verstanden werden. Alternativ kann insbesondere auch unter einem Rechner ein Knoten des verteilten Datenbanksystems verstanden werden. Mit anderen Worten kann insbesondere unter einem Gerät ein Knoten des verteilten Datenbanksystems verstanden werden oder auch ein Gerät außerhalb der Blockkette bzw. des verteilten Datenbanksystems verstanden werden. Ein Gerät außerhalb des verteilten Datenbanksystems kann beispielsweise auf die Daten (z. B. Transaktionen oder Steuertransaktionen) des verteilten Datenbanksystems zugreift und/oder von Knoten (z. B. mittels Smart-Contracts und/oder Blockketten-Orakel) angesteuert werden. Wird beispielsweise eine Ansteuerung bzw. Steuerung eines Gerätes (z. B. ein als Knoten ausgebildetes Gerät oder ein Gerät außerhalb des verteilten Datenbanksystems) durch einen Knoten realisiert, kann dies z. B. mittels eines Smart-Contracts erfolgen, der insbesondere in einer Transaktion des verteilten Datenbanksystems gespeichert ist.
[1]
   Andreas M. Antonopoulos "Mastering Bitcoin: Unlocking Digital Cryptocurrencies", O'Reilly Media, December 2014
[2]
   Roger M. Needham, Michael D. Schroeder "Using encryption for authentication in large networks of Computers" ACM: Communications of the ACM. Band 21, Nr. 12 Dezember 1978,
[3]
   Ross Anderson "Security Engineering. A Guide to Building Dependable Distributed Systems" Wiley, 2001
[4]
   Henning Diedrich "Ethereum: Blockchains, Digital Assets, Smart Contracts, Decentralized Autonomous Organizations", CreateSpace Independent Publishing Platform, 2016
[5]
   "The Ethereum Book Project/Mastering Ethereum" https://github.com/ethereumbook/ethereumbook, Stand 5.10.2017
[6]
   Leemon Baird "The Swirlds Hashgraph Consensus Algorithm: Fair, Fast, Byzantine Fault Tolerance", Swirlds Tech Report SWIRLDS-TR-2016-01, 31.5.2016
[7]
   Leemon Baird "Overview of Swirlds Hashgraph", 31.5.2016
[8]
   Blockchain Oracles https://blockchainhub.net/blockchain-oracles/ Stand 14.03.2018

Gemäß verschiedener Beispiele wird ein Verfahren zum Ausgeben einer oder mehrerer Substanzen bereitgestellt. Dabei umfasst das Verfahren folgende Schritte:
- Abspeichern eines Ausgabeplans in einer verteilten Datenbank. Der Ausgabeplan betrifft Eigenschaften oder Zielparameter der Ausgabe der Substanzen, d.h. der Ausgabeplan kann beispielsweise definieren, welche der Substanzen zu welchem Zeitpunkt und in welchem Umfang auszugeben ist. Darüber hinaus kann der Ausgabeplan alternativ oder zusätzlich beispielsweise die einzusetzende Ausgabevorrichtung (d.h. die entsprechende erfindungsgemäße Vorrichtung) und/oder das Ausgabeziel (z.B. den Patienten, einen bestimmten Behälter, einen bestimmten Ort), an welchem die mindestens eine Substanz auszugeben ist, umfassen. Anstelle eines einzelnen Zeitpunkts kann der Ausgabeplan z.B. auch allgemein einen Zeitplan definieren, nach welchem die mindestens eine Substanz auszugeben ist. Der Umfang kann etwa definieren, in welchem Niveau oder Mischverhältnis die jeweilige Substanz auszugeben ist. Darüber hinaus ist es möglich, dass der Ausgabeplan anstelle des Umfangs, in welchem die mindestens eine Substanz auszugeben ist, ein Zielniveau mindestens einer bestimmten Substanz definiert.
- Automatisches Ausgeben der Substanz oder der Substanzen in Abhängigkeit von dem Ausgabeplan. In diesem Schritt wird die Substanz bzw. werden die Substanzen insbesondere derart ausgegeben, wie es in dem Ausgabeplan definiert ist, wobei insbesondere die in dem Ausgabeplan definierten Mengen und Zeitpunkte eingehalten werden (im Rahmen der typischen Systemgenauigkeit, etwa begrenzt durch die Messgenauigkeit).
Wenn der Ausgabeplan ein Zielniveau mindestens einer bestimmten Substanz definiert, dann kann z.B. das aktuelle Niveau der mindestens einen bestimmten Substanz gemessen und insbesondere abhängig von einem Vergleich zwischen dem aktuellen Niveau und dem Zielniveau die mindestens eine Substanz ausgegeben werden. Die Techniken gemäß verschiedener Beispiele steuern in diesem Fall also quasi das Niveau der mindestens einen bestimmten Substanz durch die Ausgabe der mindestens einen Substanz.

Beispielsweise könnte der Ausgabeplan als Smart Contract im Rahmen einer Transaktion in einem oder mehreren Blöcken einer Blockkette gespeichert werden.

Indem die Substanz(en) automatisch abhängig von dem Ausgabeplan ausgegeben wird bzw. werden, kann erreicht werden, dass der Ausgabeplan besser eingehalten wird. Indem der Ausgabeplan in der verteilten Datenbank abgespeichert ist, ist die Wahrscheinlichkeit einer unberechtigten Änderung des Ausgabeplans vorteilhafterweise gering.

Unter einer Substanz wird in der vorliegenden Erfindung insbesondere ein fester, flüssiger oder gasförmiger Stoff oder ein Material verstanden.

Gemäß einer bevorzugten erfindungsgemäßen Ausführungsform umfasst das Verfahren zusätzlich ein Messen eines Niveaus einer bestimmten oder mehrerer bestimmter Substanzen. Das Ausgeben der mindestens einen Substanz wird dann abhängig von dem gemessenen Niveau ausgeführt.

Mit anderen Worten erfolgt die Ausgabe der mindestens einen Substanz beispielsweise abhängig von dem gemessenen Niveau der mindestens einen bestimmten Substanz. Dabei kann es sich bei der oder den bestimmten Substanzen, deren Niveau gemessen wird, um andere Substanzen oder (teilweise oder vollständig) um die Substanz(en) handeln, welche ausgegeben wird/werden.

Unter einem Niveau einer Substanz wird dabei insbesondere eine Konzentration dieser Substanz, ein Anteil dieser Substanz in einem Gesamtvolumen oder ein anteiliges Gewicht dieser Substanz innerhalb einer Gesamtmasse verstanden.

Das erfindungsgemäße Verfahren kann darüber hinaus ein Speichern des Ausgebens in der verteilten Datenbank im Zusammenhang mit dem Ausgabeplan umfassen.

Mit anderen Worten kann erfindungsgemäß die Tatsache, dass die mindestens eine Substanz abhängig von dem Ausgabeplan ausgegeben wurde, beispielsweise zusammen mit zusätzlichen Informationen zu der Ausgabe (z.B. die Information, dass die Ausgabe nicht möglich war) und mit einem Verweis auf den Ausgabeplan in der verteilten Datenbank abgespeichert werden. Ein solcher Verweis könnte etwa durch einen Verweis auf den Block oder die Transaktion oder den Smart Contract, der den Ausgabeplan spezifiziert, erfolgen.

Insbesondere kann das Speichern des Ausgebens in der verteilten Datenbank die Durchführung eines Vergleichs umfassen. Bei diesem Vergleich wird eine während des Ausgebens ausgegebene Menge der mindestens einen Substanz und ein Ausgabezeitpunkt, zu welchem die mindestens eine Substanz tatsächlich ausgegeben wurde, mit dem Ausgabeplan verglichen. Ein Ergebnis dieses Vergleichs wird dann in der verteilten Datenbank abgespeichert.

Bei diesem Vergleich kann insbesondere der Umfang oder die Menge jeder während des Ausgebens ausgegebenen Substanz mit dem entsprechenden Umfang bzw. der Menge, wie sie für die jeweilige Substanz in dem Ausgabeplan hinterlegt ist, verglichen werden. In der gleichen Weise kann insbesondere der Zeitpunkt, zu welchem die jeweilige während des Ausgebens ausgegebene Substanz tatsächlich ausgegeben wird, mit dem für die jeweilige Substanz im Ausgabeplan hinterlegten Zeitpunkt verglichen werden. Das in der verteilten Datenbank abgespeicherte Ergebnis des Vergleichs umfasst dann beispielsweise insbesondere die Resultate der Vergleiche der Substanzmengen und Zeitpunkte.

Anhand des Ergebnisses des Vergleichs kann vorteilhafterweise überprüft werden, wie genau der Ausgabeplan eingehalten wurde.

Gemäß einer weiteren erfindungsgemäßen Ausführungsform kann das Ausgeben der mindestens einen Substanz umfassen, dass eine Freigabe zur Ausgabe der mindestens einen Substanz erfasst wird und dass die mindestens eine Substanz nur dann ausgegeben wird, wenn vorher die Freigabe erfasst wurde, welche quasi die Ausgabe der mindestens einen Substanz freigibt.

Diese Freigabe oder Genehmigung kann dabei durch einen Menschen (z. B. durch den behandelnden Arzt), eine Validierungsvorrichtung oder ein mit der verteilten Datenbank in Beziehung stehendes Orakel (z.B. Blockkette-Orakel) erzeugt werden.

Indem die Ausgabe der mindestens einen Substanz abhängig von der Freigabe erfolgt, können vorteilhafterweise bestimmte Informationen oder Zustände berücksichtigt werden, bei denen die Ausgabe der mindestens eine Substanz anhand des Ausgabeplans zu vermeiden sind.

Die vorliegende Erfindung kann im Allgemeinen in verschiedensten Anwendungsgebieten eingesetzt werden. Nachfolgend werden nur einige Beispiele für Anwendungsgebiete genannt:
- Das erfindungsgemäße Verfahren kann in einer Fertigungsumgebung eingesetzt werden, um beispielsweise die Zusammensetzung von Fluids oder Gasen in einem Industrieprozess zu steuern.
- Mit der vorliegenden Erfindung kann eine Luft- und/oder Wasser-Qualität gesteuert werden, indem beispielsweise automatisch Reinigungsfluide und/oder Reinigungsgase abhängig von dem Ausgabeplan ausgegeben werden. Dadurch kann die vorliegende Erfindung beispielsweise in Gebäuden oder sogenannten "Smart City"-Umgebungen eingesetzt werden.
- Das erfindungsgemäße Verfahren kann zur automatischen Verabreichung von Arzneimitteln an einen Patienten eingesetzt werden.

Bei der eingesetzten verteilten Datenbank kann es sich um eine dezentralisierte Datenbank (d.h. eine Datenbank, bei welcher die Daten an verschiedenen örtlich getrennten Stellen gespeichert sind) und/oder um eine sogenannte Blockkette handeln.

Im Rahmen der vorliegenden Erfindung wird auch eine Vorrichtung zum Ausgeben einer oder mehrerer Substanzen bereitgestellt. Dabei umfasst die Vorrichtung eine Steuerung, Kommunikationsmittel, um damit eine Kommunikationsverbindung zu einer verteilten Datenbank aufzubauen, und eine Ausgabeeinrichtung, um damit die mindestens eine Substanz auszugeben. Die Vorrichtung ist ausgestaltet, um mit Hilfe der Kommunikationsmittel einen Ausgabeplan von der verteilten Datenbank zu erfassen und um die mindestens eine Substanz mit Hilfe der Steuerung abhängig von dem Ausgabeplan auszugeben. Dabei ist der Ausgabeplan gemäß der vorab beschriebenen Definition für das erfindungsgemäße Verfahren definiert.

Die Vorteile der erfindungsgemäßen Vorrichtung entsprechen im Wesentlichen den Vorteilen des erfindungsgemäßen Verfahrens, welche vorab im Detail ausgeführt sind, so dass hier auf eine Wiederholung verzichtet wird.

Bei der erfindungsgemäßen Vorrichtung kann es sich um eine von einem Patienten z. B. an einem Handgelenk tragbare Vorrichtung handeln.

Die tragbare Vorrichtung ermöglicht vorteilhafterweise, dass die mindestens eine Substanz automatisch an den Patienten (z.B. auch ohne eine Aktivität des Patienten) abhängig von dem Ausgabeplan ausgegeben wird.

Darüber hinaus kann die erfindungsgemäße Vorrichtung unter die Haut des Patienten implantierbar sein.

Mit einer solchen erfindungsgemäßen Vorrichtung kann die automatische Ausgabe der mindestens einen Substanz abhängig von dem Ausgabeplan weiter optimiert werden.

Darüber hinaus kann die erfindungsgemäße Vorrichtung Haltemittel - etwa Behälter, Hochdruckflaschen, Flaschen, usw. - umfassen, um die mindestens eine auszugebende Substanzen aufzubewahren. Dazu ist die mindestens eine Substanz insbesondere digital identifiziert, um bei der Ausgabe eine Verwechslung zu vermeiden.

Wenn der Ausgabeplan auch die Angabe des Ausgabeziels umfasst und die erfindungsgemäße Vorrichtung ausgestaltet ist, um das aktuelle Ausgabeziel zu erfassen, an welchem die Vorrichtung aktuell angebracht ist, dann kann insbesondere vor einer Ausgabe der mindestens einen Substanz eine Überprüfung erfolgen, ob das Ausgabeziel, an dem die Vorrichtung angebracht ist, auch dem Ausgabeziel gemäß Ausgabeplan entspricht. Nur wenn dies der Fall ist, wird die mindestens eine Substanz gemäß dem Ausgabeplan ausgegeben. Ansonsten wird die Ausgabe verweigert und die Verweigerung sowie der Grund für die Verweigerung in der verteilten Datenbank hinterlegt.

In weiteren Beispielen wird auch ein System zum Ausgeben einer oder mehrerer Substanzen bereitgestellt. Dabei umfasst das System beispielsweise eine Vorrichtung zum Ausgeben der mindestens einen Substanz, eine verteilte Datenbank und eine Eingabevorrichtung zur Eingabe oder Änderung eines Ausgabeplans, welcher in der verteilten Datenbank gespeichert ist bzw. zu speichern ist. Die Vorrichtung, bei der es sich um die vorab beschriebene erfindungsgemäße Vorrichtung handeln kann, ist ausgestaltet, um den Ausgabeplan von der verteilten Datenbank zu erfassen und um die mindestens eine Substanz abhängig von dem Ausgabeplan auszugeben. Dabei ist der Ausgabeplan gemäß der vorab beschriebenen Definition für das erfindungsgemäße Verfahren definiert.

Das erfindungsgemäße System umfasst in manchen Beispielen insbesondere eine Messvorrichtung, um ein Niveau mindestens einer bestimmten Substanz zu messen.

Das erfindungsgemäße System ist in manchen Beispielen insbesondere ausgestaltet, um das erfindungsgemäße Verfahren, welches vorab beschrieben worden ist, auszuführen.

Die Vorteile des erfindungsgemäßen Systems entsprechen den Vorteilen des erfindungsgemäßen Verfahrens, welche vorab im Detail ausgeführt sind, so dass hier auf eine Wiederholung verzichtet wird.

Des Weiteren beschreibt die vorliegende Erfindung ein Computerprogrammprodukt, insbesondere eine Software, welche man in einen Speicher einer programmierbaren Steuereinrichtung bzw. einer Recheneinheit einer Vorrichtung zum Ausgeben mindestens eine Substanz oder eines Systems zum Ausgeben mindestens einer Substanz laden kann. Mit diesem Computerprogrammprodukt können alle oder verschiedene vorab beschriebene Ausführungsformen des erfindungsgemäßen Verfahrens ausgeführt werden, wenn das Computerprogrammprodukt in der Steuereinrichtung läuft. Dabei benötigt das Computerprogrammprodukt eventuell Programmmittel, z.B. Bibliotheken und Hilfsfunktionen, um die entsprechenden Ausführungsformen des Verfahrens zu realisieren. Mit anderen Worten soll mit dem auf das Computerprogrammprodukt gerichteten Anspruch insbesondere eine Software unter Schutz gestellt werden, mit welcher eine der oben beschriebenen Ausführungsformen des erfindungsgemäßen Verfahrens ausgeführt werden kann bzw. welche diese Ausführungsform ausführt. Dabei kann es sich bei der Software um einen Quellcode (z.B. C++), der noch compiliert und gebunden oder der nur interpretiert werden muss, oder um einen ausführbaren Softwarecode handeln, der zur Ausführung nur noch in die entsprechende Recheneinheit bzw. Steuereinrichtung zu laden ist.

Schließlich offenbart die vorliegende Erfindung einen elektronisch lesbaren Datenträger, z.B. eine DVD, ein Magnetband, eine Festplatte oder einen USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuereinrichtung bzw. Recheneinheit einer Vorrichtung zum Ausgeben mindestens einer Substanz oder eines Systems zum Ausgeben mindestens einer Substanz gespeichert werden, können alle erfindungsgemäßen Ausführungsformen des vorab beschriebenen Verfahrens durchgeführt werden.

Die vorliegende Erfindung kann beispielsweise in folgenden Schritten (beispielsweise in der angegebenen Reihenfolge) ausgeführt werden:
- Ein qualifizierter Benutzer spezifiziert über eine Eingabevorrichtung einen Ausgabeplan für ein bestimmtes Ausgabeziel und für eine bestimmte Ausgabevorrichtung bzw. erfindungsgemäße Vorrichtung, welche an dem Ausgabeziel angebracht ist.
- Die Eingabevorrichtung speichert den Ausgabeplan unveränderbar in der verteilten Datenbank.
- Die erfindungsgemäßen Vorrichtungen überprüfen kontinuierlich, ob ihre in der verteilten Datenbank hinterlegten Ausgabepläne aktualisiert wurden.
- Basierend auf dem jeweiligen Ausgabeplan führen die erfindungsgemäßen Vorrichtungen die Ausgabe der mindestens einen Substanz für das entsprechende Ausgabeziel durch oder erzeugen einen entsprechenden Hinweis, wenn der entsprechende Ausgabeplan und die für die jeweilige erfindungsgemäße Vorrichtung verfügbaren Substanzen nicht übereinstimmen.
- Die erfindungsgemäßen Vorrichtungen speichern die Parameter der ausgeführten Schritte in der verteilten Datenbank.
- Messvorrichtungen messen jeweils das Niveau bestimmter Substanzen vom Ausgabeziel und speichern die zugehörigen Messergebnisse in der verteilten Datenbank.
- Der Ausgabeplan umfasst oder referenziert Smart Contracts in der verteilten Datenbank bzw. Blockkette. Diese Smart Contracts leiten definierte Transaktionen (z.B. die Ausgabe der mindestens einen Substanz) ein, wenn die Messergebnisse vorbestimmte Grenzwerte überschreiten oder wenn der jeweilige Ausgabeplan nicht ausgeführt werden kann.

Die vorliegende Erfindung kann beispielsweise zur Verteilung von Arzneimitteln an Patienten eingesetzt werden. Dazu definiert ein Arzt für den jeweiligen Patienten einen Ausgabeplan. Dieser Ausgabeplan, in welchem Niveaus, Mischverhältnisse und Zeitbedingungen für die Ausgabe der Arzneimittel beschrieben sind, wird als eine von diesem Arzt signierte Transaktion in der verteilten Datenbank gespeichert. Der Patient trägt eine erfindungsgemäße Vorrichtung, welche den Ausgabeplan von der verteilten Datenbank erfasst und überprüft sowie die Arzneimittel gemäß diesem Ausgabeplan an den Patienten ausgibt und die Ausgabe der Arzneimittel protokolliert und die protokollierten Ergebnisse dieser Ausgabe in der verteilten Datenbank speichert.

Die vorliegende Erfindung ermöglicht, dass beispielsweise ein qualifizierter Fachmann einen Ausgabeplan für ein bestimmtes Ausgabeziel erstellt. Beispielsweise kann ein Arzt nach einer Diagnose einen Ausgabeplan zur Ausgabe von Arzneimitteln an einen Patienten (entspricht dem Ausgabeziel) erstellen. Dieser Ausgabeplan wird unveränderlich bzw. fälschungssicher in einer verteilten Datenbank (beispielsweise als Smart Contract in einer Blockkette) hinterlegt. Eine erfindungsgemäße Vorrichtung kann dann den Ausgabeplan von der verteilten Datenbank auslesen und die Ausgabe der mindestens einen Substanz entsprechend diesem Ausgabeplan an das Ausgabeziel vornehmen. Die erfindungsgemäße Vorrichtung kann dabei alle notwendigen Vorgänge (insbesondere die Menge der ausgegebenen Substanzen und den Zeitpunkt der Ausgabe) in der verteilten Datenbank speichern.

Die vorliegende Erfindung ermöglicht vorteilhafterweise, dass eine Änderung oder Anpassung des Ausgabeplans, gerade wenn das Ausgabeziel mobil ist, in einer ganzheitlichen, nachvollziehbaren und transparenten Weise durchgeführt wird. Dabei erfolgt der Übergang von der Änderung des Ausgabeplans zu der Ausführung des Ausgabeplans zu der Überprüfung des Ausgabeplans und der Protokollierung des Ausgabeplans ohne Medienunterbrechungen.

Dabei können vorteilhafterweise alle durchgeführten Schritte in der verteilten Datenbank protokolliert werden. Das heißt, jeder durchgeführte Schritt kann nachvollzogen werden, wobei auch ermittelt werden kann, wer diesen Schritt ausgeführt hat. Fehler, welche nach dem Stand der Technik aufgrund von Medienwechseln auftreten, können vorteilhafterweise vollständig vermieden werden.

Jeder in dem Ausgabeplan bezüglich der Ausgabe der mindestens einen Substanz hinterlegte Schritt führt vorteilhafterweise zu einer garantierten Ausführung.

Ein Missbrauch oder eine Fehlanwendung ist nahezu unmöglich, da jede Ausgabe durch signierte Transaktionen in der verteilten Datenbank hinterlegt wird.

Die Erfindung ermöglicht darüber hinaus, dass die Qualifikation von Rollen und Teilnehmern anhand derselben verteilten Datenbank in einer dezentralen Weise vorgenommen werden kann, um sicherzustellen, dass nur qualifizierte Teilnehmer (z. B. ein Arzt, ein verantwortliches Bedienpersonal, eine zertifizierte Ausgabevorrichtung), welche durch entsprechende Autoritäten zertifiziert worden sind, einen Ausgabeplan ausführen und/oder erstellen bzw. ändern können. Der Prozess zum Erlangen einer bestimmten Rolle kann auch in die vorliegende Erfindung aufgenommen werden.

### KURZE BESCHREIBUNG DER FIGUREN

Im Folgenden wird die vorliegende Erfindung anhand erfindungsgemäßer Ausführungsformen mit Bezug zu den Figuren im Detail beschrieben.
- In Fig. 1: ist schematisch ein erfindungsgemäßes System im Zusammenspiel mit einer Blockkette dargestellt.
- In Fig. 2: ist schematisch der Flussplan eines erfindungsgemäßen Verfahrens zum Ausgeben mindestens einer Substanz abgebildet.

### DETAILLIERTE BESCHREIBUNG VON AUSFÜHRUNGSFORMEN

Das erfindungsgemäße System 20 umfasst eine erfindungsgemäße Vorrichtung 9, eine verteilte Datenbank 10, eine Eingabevorrichtung 4 und eine Messvorrichtung 5. Die Eingabevorrichtung 4 ist ausgestaltet, um einen Ausgabeplan einzugeben oder zu ändern, welcher in der verteilten Datenbank 10 gespeichert ist. Dazu umfasst die Eingabevorrichtung 4 insbesondere Kommunikationsmittel, um eine Kommunikationsverbindung zu der verteilten Datenbank 10 aufzubauen. Die erfindungsgemäße Vorrichtung 9 umfasst neben einer Steuerung 2 Kommunikationsmittel 3, um mit diesen eine Kommunikationsverbindung zu der verteilten Datenbank 10 aufzubauen, und eine Ausgabeeinrichtung 1, um mindestens eine Substanz auszugeben. Zusätzlich kann die erfindungsgemäße Vorrichtung 9 die Messvorrichtung 5 umfassen, welche ein Niveau einer oder mehrerer bestimmter Substanzen misst.

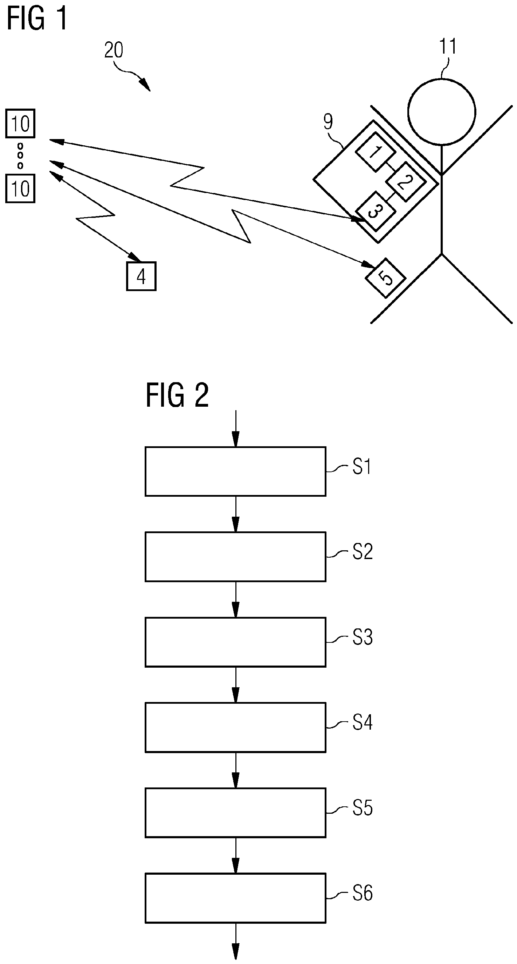

Wie man dem Beispiel der Fig. 1 entnehmen kann, wird die erfindungsgemäße Vorrichtung 9 von einem Patienten 11 getragen. Dadurch ist die erfindungsgemäße Vorrichtung 9 vorteilhafterweise in der Lage, die mindestens eine Substanz automatisch beispielsweise in eine Vene des Patienten 11 zu injizieren. Auch die Messvorrichtung 5 ist an dem Patienten 11 befestigt, um so das Niveau der mindestens einen bestimmten Substanz (z.B. der Blutzuckerkonzentration) bei dem Patienten 11 automatisch zu messen. Abhängig von den mittels der Messvorrichtung 5 gemessenen Messergebnissen kann dann die mindestens eine Substanz abhängig von dem Ausgabeplan ausgegeben werden. Die Messergebnisse übermittelt die Messvorrichtung 5 entweder selbst direkt an die verteilte Datenbank 10 oder, wenn die Messvorrichtung 5 Bestandteil der erfindungsgemäßen Vorrichtung 9 ist, über die Kommunikationsmittel 3 der Vorrichtung 9 an die verteilte Datenbank 10.

In Fig. 2 ist der Flussplan eines erfindungsgemäßen Verfahrens zum Ausgeben mindestens einer Substanz schematisch dargestellt.

Im ersten Schritt S1 wird ein Ausgabeplan erstellt und in einer verteilten Datenbank gespeichert. Im zweiten Schritt S2 wird das Niveau einer bestimmten Substanz gemessen. Die Ergebnisse dieser Messung können auch in der verteilten Datenbank hinterlegt werden. Im dritten Schritt S3 wird die Freigabe zur Ausgabe einer oder mehrerer Substanzen erfasst. Nur wenn diese Freigabe vorher erfasst wurde, wird im vierten Schritt S4 mindestens eine Substanz unter Berücksichtigung des in der verteilten Datenbank abgespeicherten Ausgabeplans und unter Berücksichtigung der in Schritt S2 erzielten Messergebnisse ausgegeben. Daher umfasst der Schritt S4 ein Auslesen des Ausgabeplans und der Messergebnisse aus der verteilten Datenbank. Im folgenden Schritt S5 wird die tatsächlich ausgegebene Menge der mindestens einen Substanz und der Zeitpunkt, zu welchem die mindestens eine Substanz ausgegeben wurde, mit dem Ausgabeplan verglichen. Die Ergebnisse dieses Vergleichs werden im folgenden Schritt S6 in der verteilten Datenbank abgelegt.

Nachdem die Schritte S2 bis S6 mehrfach durchgeführt wurden, kann durch eine Auswertung der in der verteilten Datenbank gespeicherten Daten ermittelt werden, wie genau der Ausgabeplan befolgt wurde.

## Patentansprüche

1. Verfahren zum Ausgeben mindestens einer Substanz,
wobei das Verfahren folgende Schritte umfasst:
Speichern eines Ausgabeplans in einer verteilten Datenbank (10), wobei der Ausgabeplan definiert, welche der mindestens einen Substanz zu welchem Zeitpunkt und in welcher Menge auszugeben ist, und
Ausgeben der mindestens einen Substanz abhängig von dem Ausgabeplan.

2. Verfahren nach Anspruch 1,
**dadurch gekennzeichnet,**
**dass** das Verfahren zusätzlich umfasst
Messen eines Niveaus mindestens einer bestimmten Substanz, und
**dass** das Ausgeben der mindestens einen Substanz abhängig von dem Niveau ausgeführt wird.

3. Verfahren nach Anspruch 1 oder 2,
**dadurch gekennzeichnet,**
**dass** das Verfahren weiterhin umfasst:
Speichern des Ausgebens in der verteilten Datenbank im Zusammenhang mit dem Ausgabeplan.

4. Verfahren nach Anspruch 3,
**dadurch gekennzeichnet,**
**dass** das Speichern des Ausgebens in der verteilten Datenbank umfasst:
Durchführen eines Vergleichs einer während des Ausgebens ausgegebenen Menge der mindestens einen Substanz und einem Ausgabezeitpunkt mit dem Ausgabeplan, und
**dass** ein Ergebnis des Vergleichs in der verteilten Datenbank (10) gespeichert wird.

5. Verfahren nach einem der vorhergehenden Ansprüche, **dadurch gekennzeichnet,**
**dass** das Ausgeben der mindestens einen Substanz umfasst, dass eine Freigabe zur Ausgabe der mindestens einen Substanz erfasst wird und dass die mindestens eine Substanz nur ausgegeben wird, wenn vorher die Freigabe erfasst wurde.

6. Verfahren nach einem der vorhergehenden Ansprüche, **dadurch gekennzeichnet,**
**dass** das Verfahren in einer Fertigungsumgebung eingesetzt wird, oder
**dass** das Verfahren zur Reinerhaltung von Luft und/oder Wasser eingesetzt wird, oder
**dass** das Verfahren zur automatischen Verabreichung von Arzneimitteln an einen Patienten (11) eingesetzt wird.

7. Verfahren nach einem der vorhergehenden Ansprüche, **dadurch gekennzeichnet,**
**dass** die verteilte Datenbank dezentralisiert ist, und/oder dass die verteilte Datenbank als eine Blockkette (10) realisiert ist.

8. Vorrichtung zum Ausgeben mindestens einer Substanz,
wobei die Vorrichtung (9) eine Steuerung (2), Kommunikationsmittel (3) zum Aufbauen einer Kommunikationsverbindung zu einer verteilten Datenbank (10) und eine Ausgabeeinrichtung (1) zum Ausgeben der mindestens einen Substanz umfasst,
wobei die Vorrichtung (9) ausgestaltet ist, um mittels der Kommunikationsmittel (3) einen Ausgabeplan von der verteilten Datenbank (10) zu erfassen und um die mindestens eine Substanz mittels der Steuerung (2) abhängig von dem Ausgabeplan auszugeben, wobei der Ausgabeplan definiert, welche der mindestens einen Substanz zu welchem Zeitpunkt und in welcher Menge auszugeben ist.

9. Vorrichtung nach Anspruch 8,
**dadurch gekennzeichnet,**
**dass** die Vorrichtung (9) als eine von einem Patienten (11) tragbare Vorrichtung ausgestaltet ist.

10. Vorrichtung nach Anspruch 8 oder 9,
**dadurch gekennzeichnet,**
**dass** die Vorrichtung (9) unter die Haut eines Patienten (11) implantierbar ist.

11. Vorrichtung nach einem der Ansprüche 8 bis 10,
**dadurch gekennzeichnet,**
**dass** die Vorrichtung (9) zur Durchführung des Verfahrens nach einem der Ansprüche 1-7 ausgestaltet ist.

12. System zum Ausgeben mindestens einer Substanz,
wobei das System (20) eine Vorrichtung (9) zum Ausgeben der mindestens einen Substanz, eine verteilte Datenbank (10) und eine Eingabevorrichtung (4) zur Eingabe oder Änderung eines Ausgabeplans, welcher in der verteilten Datenbank (10) gespeichert ist, umfasst,
wobei die Vorrichtung (9) ausgestaltet ist, um den Ausgabeplan von der verteilten Datenbank (10) zu erfassen und um die mindestens eine Substanz abhängig von dem Ausgabeplan auszugeben, wobei der Ausgabeplan definiert, welche der mindestens einen Substanz zu welchem Zeitpunkt und in welcher Menge auszugeben ist.

13. System nach Anspruch 12,
**dadurch gekennzeichnet,**
**dass** das System (20) eine Messvorrichtung (5) umfasst, um ein Niveau mindestens einer bestimmten Substanz zu messen.

14. System nach Anspruch 12 oder 13,
**dadurch gekennzeichnet,**
**dass** die Vorrichtung die Vorrichtung (9) nach einem der Ansprüche 8 bis 11 ist.

15. Computerprogrammprodukt, welches ein Programm umfasst und direkt in einen Speicher einer programmierbaren Steuerung einer Vorrichtung (9) zum Ausgeben mindestens einer Substanz oder eines Systems (20) zum Ausgeben mindestens einer Substanz ladbar ist, mit Programm-Mitteln, um alle Schritte des Verfahrens nach einem der Ansprüche 1-7 auszuführen, wenn das Programm in der Steuerung ausgeführt wird.
